# EUROPEAN PATENT SPECIFICATION

(11) **EP 3 508 583 B1**
(45) Date of publication and mention of the grant of the patent: **31.08.2022**
(21) Application number: 18248212.5
(22) Date of filing: 28.12.2018
(51) Int. Cl.: C12P 5/00, C12M 1/00, C12M 1/107, C10G 1/00, C10G 45/02, C10G 45/58, C10L 1/02, C10L 1/04

(54) **METHOD FOR OBTAINING INTEGRATED BIOREFINERIES OF LIGNOCELLULOSE BIOMASSES BY REUSING PRODUCTION UNITS OF FORMER OIL REFINERIES**
VERFAHREN ZUR HERSTELLUNG INTEGRIERTER BIORAFFINERIEN VON LIGNOCELLULOSEBIOMASSE DURCH WIEDERVERWENDUNG VON PRODUKTIONSEINHEITEN EHEMALIGER ÖLRAFFINERIEN
PROCÉDÉ D'OBTENTION DE BIORAFFINERIES INTÉGRÉES DE BIOMASSES DE LIGNOCELLULOSE EN RÉUTILISANT DES UNITÉS DE PRODUCTION D'ANCIENNES RAFFINERIES DE PÉTROLE

(30) Priority: 03.01.2018 IT 201800000312
(43) Date of publication of application: 10.07.2019
(73) Proprietor: Water & Soil Remediation S.r.l., 46010 Curtatone, Frazione Levata MN (IT)
(72) Inventor: PRANDI, Alberto, 46100 Mantova (IT)
(74) Representative: Modiano, Micaela Nadia

(56) References cited:
- WO-A1-2014/033762
- WO-A2-2010/144684
- eni Green Refinery Indd: "Green Refinery: reinventing petroleum refineries", , 23 May 2014 (2014-05-23), 2 September 2014 (2014-09-02), XP055465315, Retrieved from the Internet: URL:https://www.eni.com/docs/en_IT/enicom/ publications-archive/company/operations-st rategies/refining-marketing/eni_Green-Refi nery_esecutivo.pdf [retrieved on 2018-04-06]
- JUAN ANTONIO MELERO ET AL: "Biomass as renewable feedstock in standard refinery units. Feasibility, opportunities and challenges", ENERGY & ENVIRONMENTAL SCIENCE, vol. 5, no. 6, 2012, page 7393, XP055046864, ISSN: 1754-5692, DOI: 10.1039/c2ee21231e
- MAHMOUD A ET AL: "Systematic methodology for optimal enterprise network design between bio-refinery and petroleum refinery for the production of transportation fuels", ENERGY, vol. 59, 2013, pages 224-232, XP028708853, ISSN: 0360-5442, DOI: 10.1016/J.ENERGY.2013.06.026
- Marcus Vinicius de Oliveira Magalhães: "Integrating Refining to Petrochemical" In: Rita Maria de Brito Alves, Claudio Augusto Oller do Nascimento and EvaristoChalbaud Biscaia Jr.: "10th International Symposium on Process Systems Engineering - PSE2009", 2009, Elsevier pages 107-112,

## Description

The present invention relates to a method for obtaining integrated biorefineries of lignocellulose biomasses which biorefineries transform said lignocellulose biomasses into products for food, industrial, energy-related, and agricultural use .

As is known, the transformation of lignocellulose biomasses can be obtained by means of both thermochemical and biochemical processes.

Known biochemical processes essentially provide: a physical pretreatment of the raw material, a step of enzymatic hydrolysis of the celluloses, that is optionally preceded by the extraction of the oligo-mono-hemicelluloses, the transformation of sugars into various substances useful to the market, the separation of the lignin part and any processing thereof.

It is also known that methods have been proposed which allow the conversion of parts of former oil refineries in order to obtain plants capable of performing the biorefining of lignocellulose products.

There is, for example, the case of a former oil refinery located on Italian territory, in which two units, previously operating to perform hydrodesulfurization of oil products, have been transformed in order to provide the hydrodeoxygenation of lipidic biomass and the subsequent isomerization of the resulting paraffinic hydrocarbon, in order to obtain biofuels.

A project is also known for converting one or more plants present in another former Italian oil refinery, in order to obtain the transformation of the organic fraction of municipal solid waste (OFMSW) and of other biomasses into biofuels, according to thermochemical processes.

Other known methods provide for the processing in oil refineries of vegetable oils mixed with hydrocarbons, in order to obtain mixtures of fuels of mixed fossil and biological origin.

Generally, biorefineries obtained by reusing portions of former oil refineries operate on the basis of thermochemical processes, in which the transformations occur at high temperatures, by hydrogenation, cracking and/or thermal or catalytic hydrocracking.

Processes of the thermochemical type on materials of biological origin are characterized by significant energy consumption, requiring temperatures and pressures of a certain level and consequently requiring to keep ovens and boilers in operation, with significant consumptions of fuels and emissions of combustion gases in the atmosphere.

Another limitation of thermochemical transformations resides in that the finished products obtained have compositions which are very similar, if not identical, to those of hydrocarbons; therefore they can have exclusively energy-related destinations, for example as combustibles or fuels, with very few exceptions.

As a result, the cost-effectiveness of biorefineries based on thermochemical transformations depends on energy prices, and this exposes these biorefineries to vulnerability due to the price oscillations that oil products undergo over time, as demonstrated by the numerous energy crises that have occurred in the last half century.

WO2014033762 discloses a method for revamping a conventional refinery of mineral oils into a biorefinery, characterized by a production scheme which allows the treatment of raw materials of a biological origin (vegetable oils, animal fats, exhausted cooking oils) for the production of biofuels, prevalently high-quality biodiesel.

WO2010144684 discloses a process for preparing fuels, such as diesel fuels or jet fuels, by hydrotreating vegetable oils or fatty acid derivatives that may be applied to existing equipment for treating fossil fuels.

Eni Green Refinery Indd, "Green Refinery: reinventing petroleum refineries", (20140523), URL: https://www.eni.com/docs/en_IT/enicom/publications-archive/company/operations-strategies/refining-marketing/eni_Green-Refinery_esecutivo.pdf, teaches the conversion of a conventional petroleum refinery into a biorefinery.

JUAN ANTONIO MELERO ET AL, "Biomass as renewable feedstock in standard refinery units. Feasibility, opportunities and challenges", ENERGY & ENVIRONMENTAL SCIENCE, (2012), vol. 5, no. 6, discusses the possibility of production of biofuels (biorefining) in standard oil refining units.

MAHMOUD A ET AL, "Systematic methodology for optimal enterprise network design between bio-refinery and petroleum refinery for the production of transportation fuels", ENERGY, (2013), vol. 59, teaches the integration of biorefinery starting from lignocellulosic biomass of oils in a petroleum refinery plant.

The aim of the present invention is to provide a method for obtaining biorefineries by reusing production units of former oil refineries selected from a distillation unit, a conversion unit, storage units, a desulfurization unit, a catalytic isomerization unit, and a gas scrubbing unit, that is capable of improving the background art in one or more of the above mentioned aspects.

Within this aim, an object of the invention is to provide a method that allows to obtain biorefineries of lignocellulose biomasses with a production plan with low investment but high diversification of processes and finished products.

Another object of the invention is to provide a method that makes it possible to reuse a large part of former oil plants with few modifications, thus keeping to a minimum the investment costs of new biorefineries.

Another object of the invention is to provide a method that allows to obtain biorefineries capable of transforming lignocellulose biomasses and other second- and third-generation biomasses into products for food, industrial, energy-related and agricultural use, according to a flexible method that entails mutually harmonized processes and reuses.

A further object of the present invention is to overcome the drawbacks of the background art in a manner that is alternative to any existing solutions.

Another object of the invention is to provide a method that is highly reliable, relatively easy to provide and at competitive costs.

### Summary of the invention

This aim and these and other objects which will become better apparent hereinafter are achieved by a method according to claim 1, optionally provided with one or more of the characteristics of the dependent claims 2-7.

### Detailed description of the invention

Further characteristics and advantages of the invention will become better apparent from the description of some preferred but not exclusive embodiments of the method according to the invention, illustrated by way of non-limiting example in the accompanying drawings, wherein:
Figure 1 is a schematic view of various production units of which an oil refinery is typically composed;
Figure 2 is a schematic view of a biorefinery that can be obtained by means of the method according to the invention;
Figures 3, 4 and 5 are schematic views of various examples of reuse of tanks of former oil refineries according to the proposed method. With reference to Figure 1, various production units, also termed production plants or simply plants or units, that typically compose a traditional oil refinery, generally designated by 100, are described hereinafter.

In particular, in a traditional oil refinery there are one or more storage units, constituted by initial storage tanks 1, from which the raw material, for example constituted by crude oil, feeds a first distillation unit 2, constituted for example by a distillation column, which produces different fractions, such as, for example, light distillates, which exit from the first distillation unit 2 by means of a first line 3, medium distillates, which exit from the first distillation unit 2 by means of a second line 4, and a column bottom residue, which exits from the first distillation unit 2 by means of a third line 5.

The light distillates are subjected to a catalytic hydrodesulfurization treatment and to further fractionation in a first desulfurization unit 6, as well as subsequent processes.

In particular, the exhaust gases in output from the first desulfurization unit 6 can be sent to a gas scrubbing unit 7, in which they are separated from the hydrogen sulfide gas (H₂S) generated by the hydrodesulfurizations, to an LPG treatment and separation unit 8, from which the commercial fractions propane and butane are obtained, to a catalytic isomerization unit 9 for the hydrocarbons pentanes and hexanes, and to a catalytic reforming unit 10 of the desulfurized virgin naphtha cut. The products in output from the catalytic isomerization and catalytic reforming units 9 and 10 are subjected to an intermediate and final storage in storage units, constituted by intermediate and final storage tanks 11.

The medium distillates undergo a treatment of catalytic hydrodesulfurization in a second desulfurization unit 12, with further production of acid gas due to the presence of hydrogen sulfide (H₂S), which is sent to the gas scrubbing unit 7.

The bottom residues of the first distillation unit 2 are subjected to further processes in conversion units 13 in order to increase their value through their transformation into high-value products such as light or medium distillates or also bitumens, which also enjoy a good added value market.

The conversion processes to which the bottom residues are subjected in the conversion units 13 can be thermal or catalytic cracking, thermal or catalytic hydrocracking, hydrocracking of components thereof such as vacuum distillates, their gasification, with almost total disappearance of the residue, i.e., of the fuel oil, as well as further cascade processes in other processing units 14. In particular, the conversion units 13 comprise reactors, fractionation columns, systems for managing pressures and temperatures, in a manner similar to other production units.

All the products in output from the plants of the refinery, such as gasolines, kerosenes, diesel fuels, fuel oils, bitumens and so on, are poured into storage units, constituted by intermediate and final storage tanks 11, for blending operations and finally for selling on the market.

Both the initial storage tanks 1 and the intermediate and final storage tanks 11 are numerous in every oil refinery and moreover they have a wide assortment in terms of volume capacity and roof structure, which can be fixed or floating. For example, the initial storage tanks 1 can have a high volumetric capacity and a floating roof, while the intermediate and final storage tanks 11 are less voluminous than the preceding ones and have significant variability both as capacity and as type of roof.

With respect to the typical crude oil refining process plan described above, in the oil sector there are plan and plant solution variations which are tailored for each individual situation. For example, the LPG fractions can be separated and desulfurized separately from the other fractions; the gas scrubbing units 7 are often more than one and so are the hydrodesulfurization units; the column bottom residue conversion units 13 can differ in terms of operating conditions and process (thermal cracking, catalytic cracking, catalytic cracking with hydrogen, continuous fluid bed cracking, etc.) and can be with or without vacuum distillation.

In any case, the basic plan of an oil refinery is always composed of some fundamental units, such as for example the first distillation unit 2, the light distillate desulfurization unit 6, the medium distillate desulfurization unit 12, the gas scrubbing units 7, various units 13 for the treatment of the column bottom residue, with or without vacuum distillation.

Various units of a disused oil refinery with this basic plan, comprising also storage tanks 1 and 11 and surrounding equipment, can be the subject of reuse according to the present invention, in order to obtain a biochemical biorefinery that is organized and integrated in diversified productions, at minimum investment cost, as described hereinafter.

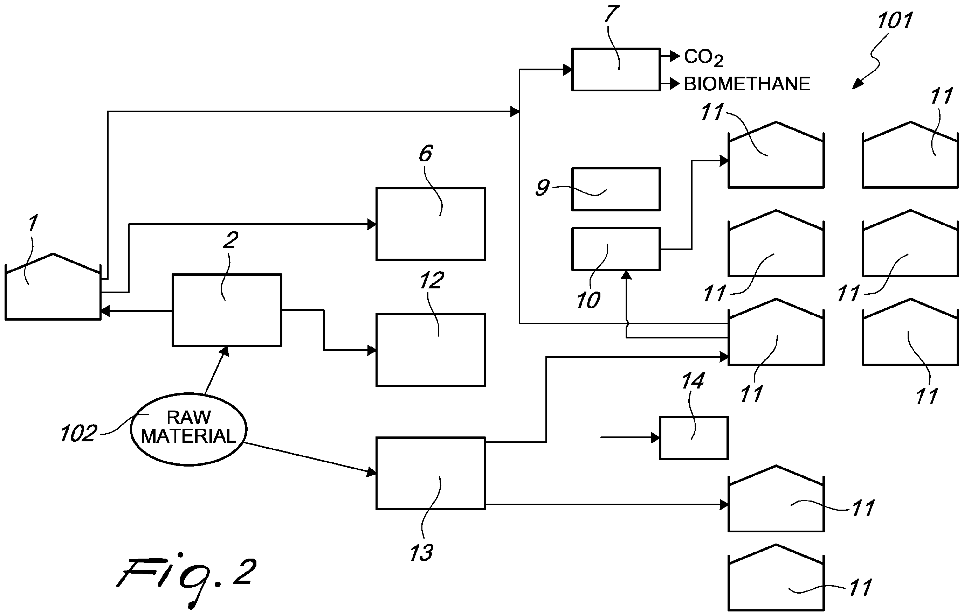

Figure 2 shows, by way of example, that at least some production units of an oil refinery can be reused, according to the method of the present invention, in order to obtain a biorefinery capable of performing the biochemical biorefining of biomasses and particularly of lignocellulose biomasses.

In particular, the first distillation unit 2 has the equipment useful to perform a pretreatment of the initial raw material, constituted by lignocellulose biomasses, by means of a steam explosion or equivalent process, using a distillation column as a flash vessel, heating systems and heat exchange systems, temperature and pressure adjustment systems; other production units of the refinery, such as for example the conversion units 13, also provided substantially with similar equipment, are also suitable for the same purpose and therefore reusable.

Some equipment that is part of the processing units 14 that in the oil refinery were used to treat the residues of the treatments performed by the conversion unit 13 can be reused for the treatment of lignin residues.

Thus, for example, distillation columns and reactors of the previous refinery can be reused, as part of the new biorefinery, in order to perform a pretreatment of the initial raw material, i.e., of the lignocellulose biomass.

This pretreatment may be at atmospheric pressure, according to a process of the steam explosion type, and/or at a pressure lower than the atmospheric pressure, according to a process of the vacuum flash type.

More particularly, for this purpose, a former distillation column may be reused so as to receive inside it, through the supply line, a mixture formed by the lignocellulose biomass to be pretreated and steam, or by the lignocellulose biomass to be pretreated and preheated and pressurized water, so as to cause inside the distillation column the sudden or flash expansion of the mixture, respectively at ambient pressure or at a pressure lower than the ambient pressure, and in this case the distillation column is connected to vacuum generation means.

The storage units of the previous oil refinery and particularly the storage tanks 1 and 11 may instead be reused as part of the new biorefinery in order to provide biochemical reactions (fermentations), since they can contain the organic reagents for the times required to complete said reactions.

Optionally, in order to adapt them to the new use, the storage tanks 1 and 11 may be thermally insulated, by means of thermal insulation layers, located for example externally, and provided with thermostat-based control systems 15, such as to maintain the desired temperatures.

There are many reactions and consequent products obtainable from the organic substances contained in lignocellulose biomasses: saccharides with 5 or 6 carbon atoms, oligomers or monomers, alcohols, ketones, ethers, organic acids, various ether compounds, biogases, biomethane, carbon dioxide, polyphenols and countless other organic compounds.

Also according to the proposed method, the production unit constituted by the first desulfurization unit 6, which in the oil refinery was used for the hydrodesulfurization of the light crude fractions and for fractionating them, may be reused in the biorefinery to hydrogenate oligo- and monosaccharides and to produce other substances useful for mankind, by virtue of the fact that is provided with a hydrogenation reactor (after replacement of the catalyst with a type adapted for the purpose), compressors for make-up and recycled hydrogen, fractionation columns, temperature and pressure adjustment systems.

Other units of the previous oil refinery are suitable for the same purpose and therefore are also reusable in the biorefinery, for example the second desulfurization unit 12, which in the oil refinery was used for the catalytic hydrodesulfurization of the medium distillates, as well as the catalytic isomerization unit 9, because they too are provided with the same equipment as the first desulfurization unit 6.

Numerous production units of the oil refinery and, particularly, all those provided with a fractionation column, such as the first distillation unit 2, as well as the fractionation part of the units 6, 10, 12 and 13, can be reused, according to the invention, in order to distill, in the biorefinery, soups or alcoholic compounds, obtained as fermentation products, in order to obtain the given organic compound in concentrated form, for example the alcohol concentrated in the column head.

The production unit constituted by the gas scrubbing unit 7, which in traditional oil refineries is designed to remove the hydrogen sulphide from the fuel gas by means of amines in an aqueous solution, can be reused to separate the carbon dioxide from the biogas and obtain biomethane.

It should be added that according to the proposed method, any units for the self-generation of electric power and heat of a former oil refinery may be, advantageously, reused in the biorefinery in order to meet the enthalpic and electrical demands of the new processes.

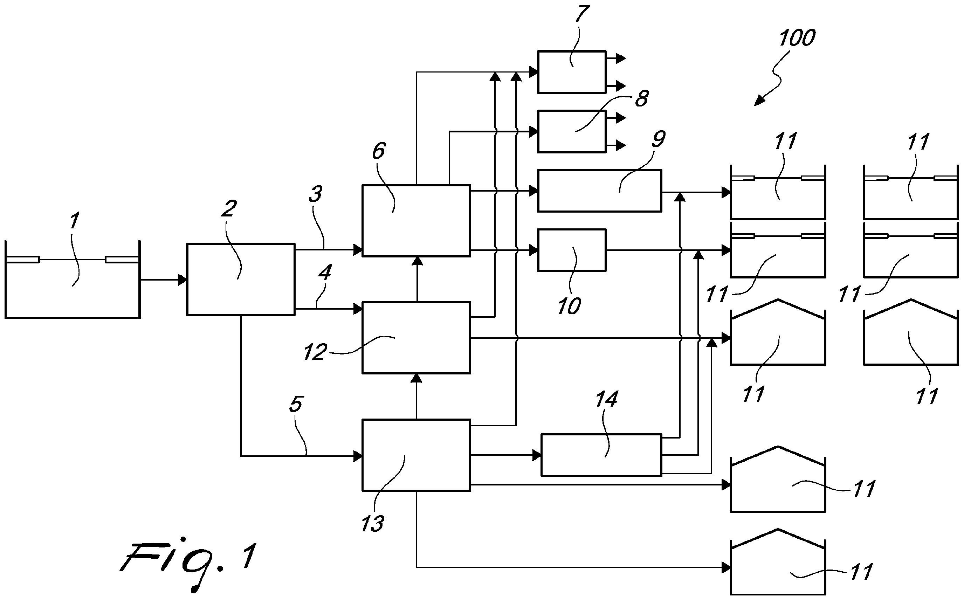

From what has been described it is evident that since all the oil refineries have production units having functions which are identical or at least similar to those exemplified in Figure 1, for the vast majority and at least for their main components, these units can be reused to provide a biorefinery capable of biorefining lignocellulose biomasses with biochemical methods, with the result of being able to obtain a considerable containment of investment costs with respect to the background art, and this is a first considerable advantage of the present invention.

A further important aspect of the invention resides in that it entails that the biorefinery obtained by means of the reuse of production units of a previous disused oil refinery is structured in different working sections, capable of operating according to a sequence of operating steps that provide for the separation of the three main components of lignocelluloses, i.e., hemicellulose, cellulose and lignin, as free as possible from chemicals and degradations, in order to be able to subsequently subject them to processes of high added value.

In particular, the method according to the invention provides for structuring the obtained biorefinery so that it can perform: a pretreatment of the lignocellulose biomass with exclusively physical-mechanic methods, which can for example comprise the mixing of the lignocellulose biomass with superheated water and/or with water vapor, the prolonged heating of the mixture under pressure, the sudden expansion of the mixture at atmospheric pressure or in vacuum; extraction of the hemicellulose after the pretreatment; saccharification of the celluloses and separation, for example by filtration and/or centrifugation, the insoluble lignin part, intended for subsequent transformations, leaving the saccharides to the different fermentation and non-fermentation processes.

It should be noted that while hemicellulose depolymerizes immediately during the pretreatment and becomes soluble, cellulose requires saccharification (by transforming into soluble sugars) and only then is it possible to separate from it the lignin part, which remains insoluble.

Conveniently, the lignins separated from the saccharides may be subjected to various transformation methods, by means of which it is possible to obtain vanillin or a secondary raw material for the production of bioplastic or terephthalic acid or paraxylene. Alternatively, the lignins may be used as fuel.

As indicated above, these treatments of the lignocellulose biomasses will be performed in preexisting production units of previous oil refineries, which according to the proposed method are reused for the vast majority of the corresponding apparatuses, thus minimizing the investment costs of the new biorefinery.

It should be noted that the separation of hemicelluloses, lignin and saccharides gives broad operational flexibility to the subsequent processes.

This flexibility is particularly important for celluloses, since the separation of lignin residues from oligo- and monosaccharides allows their best recovery; these for example can be subjected to the most disparate processes, both fermentative (ethanol, isobutanol and alcohol processes of another type, acetone and ketone processes of another type, levulinic acid processes and organic acid processes of another type, methane processes, etcetera), and chemical (for example, with hydrogenation of xylose to obtain xylitol), without interference from lignins or other residues.

These processes on saccharides are, according to the invention, performed by using the tanks 1 and 11 of a disused oil refinery, which will be chosen according to the characteristics, for example with floating or fixed roof, and/or the volumetric capacities, which depend on the necessary residence times required by each single reaction.

These processes are flexible, in the sense that they can be aimed toward obtaining one or more products instead of others, according to the market requirements, which vary over time. In order to obtain this flexibility, in the biorefinery, once saccharification of the celluloses and clarification of the resulting soluble sugars are completed, the soluble sugars can be sent towards a plurality of different fermentations, by virtue of the provision of a certain number of tanks 1 and 11, conveniently chosen to act as fermentation reactors. It is believed that this is one of the prerogatives of the invention.

The advantage of reusing the tanks 1 and 11 as fermentation reactors for the saccharides without the solid lignin residues is that it is possible to feed these tanks with sugar syrups that have undergone clarification and lack insoluble substances which, by precipitating inside them, would produce accumulations on the bottom interfering with the biochemical reactions and would be expensive due to the periodic cleaning that they would require.

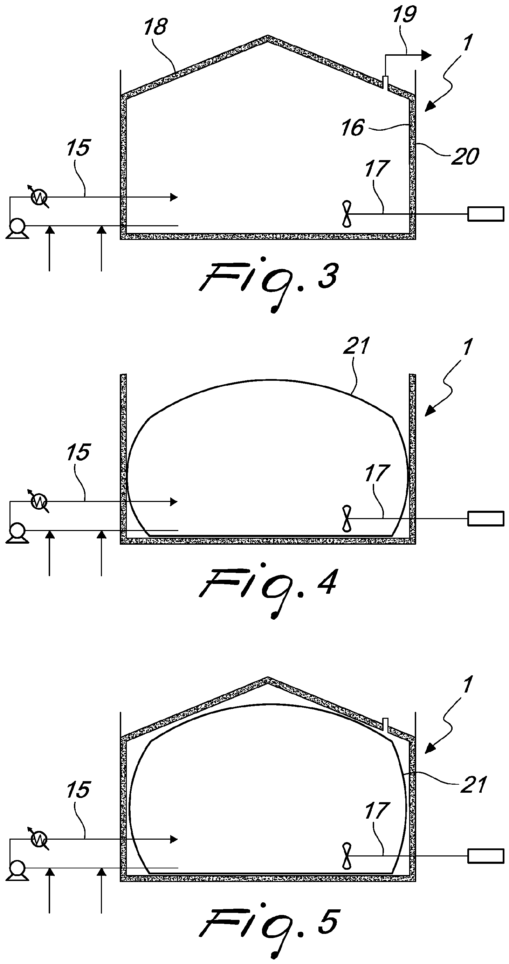

As described earlier, inside the storage base of each oil refinery there is a wide possibility of choice among different types of tanks, as a function of the required characteristics; each tank reused for the new biochemical function is subjected to a retrofit, consisting in general cleaning, inner lining compatible with the new reaction environment, optional modification of the roof, optional enhancement of the hermetic seal, thermal insulation and thermostat control, optional application of internal stirrer-mixers, as indicated in Figures 3, 4, and 5.

More particularly, since each biochemical process requires its own reaction environment, in terms of sterility from extraneous biological agents, pH, temperature, mixing, doses, residence times, corrosion resistance, the tanks 1 and 11, with fixed roof or with floating roof, that are reused may be subjected to appropriate adaptations to the new function of fermentation reactors.

In particular, the example of Figure 3 indicates some modifications that can be made to an existing tank with fixed roof or with floating roof in order to make it suitable for its reuse in the new function of fermentation reactor. Conveniently, it is possible to provide for the application of an inner lining 16 to the existing tank body, impervious to the biochemical reaction environment, of stirrer-mixers 17 of the reacting medium, of a roof 18, if the tank was not provided with one, of ports 19 for extracting the fermentation gases in output from the roof, as well as of an insulating layer 20 of the tank and a system 15 for thermostat-based control of the reacting medium, as mentioned also previously.

The example of Figure 4 indicates some modifications that can be made to a preexisting tank with floating roof in order to make it suitable for the new function of fermentation reactor, among which the application of an inner flexible enclosure 21, with a capacity approximately equal to the tank itself, also with a double chamber, designed to contain the fermenting mass, is highlighted.

Example 5 indicates instead the application of a flexible enclosure 21 inside a tank with a fixed roof, with a capacity approximately equal to the tank itself, also with a double chamber.

Again with reference to Figure 2, a possible diagram of a biorefinery obtainable by means of the proposed method, generally designated by 101, can provide for reuse of a first distillation unit 2 and a conversion unit 13 of a disused oil refinery in order to provide a pretreatment of distillation of the lignocellulose biomass 102 fed in input to the biorefinery. This pretreatment allows to extract hemicelluloses and celluloses to be subjected to subsequent treatments, freeing them form the confinement performed by the lignins, to obtain their saccharification and finally the separation of the lignins.

The first distillation unit 2, thus reused, is connected, for example, to a desulfurization unit 12 of a former oil refinery, reused to perform a catalytic hydrogenation, and to an initial storage tank 1, reused as fermentation reactor of the products in output from the unit 2, particularly soluble sugars, and, in turn, connected in output to a previous desulfurization unit 6, reused to perform a catalytic hydrogenation, as well as to a gas scrubbing unit 7 of a former oil refinery, reused to separate the carbon dioxide CO₂ from the biogas produced by fermentation, in order to obtain biomethane.

Advantageously, the conversion unit 13 reused to perform the pretreatment of the lignocellulose biomass may be connected in output to one or more intermediate or final storage tanks 11 of a former oil refinery, reused as fermentation reactors capable of performing different types of fermentation so as to be able to obtain different products. In turn, the tanks 11 used as fermentation reactors may be connected in output to the gas scrubbing unit 7 (for example in order to separate carbon dioxide from biomethane) and/or to a catalytic reforming unit 10 of the previous oil refinery, reused to perform the distillation of the aqueous solution after fermentation, the output of which is connected to other tanks 11 of the previous oil refinery, reused for storage of the final products.

From the above it is evident that the invention is able to achieve the intended aim and objects.

The invention thus conceived is susceptible of numerous modifications and variations, within the scope of the appended claims.

In practice, the materials used, so long as they are compatible with the specific use, as well as the contingent shapes and dimensions, may be any according to the requirements and the state of the art.

## Claims

1. A method for obtaining integrated biorefineries of lignocellulose biomasses which biorefineries transform said lignocellulose biomasses into products for food, industrial, energy-related, and agricultural use, **characterized in that**
production units of former oil refineries selected from a distillation unit (2), a conversion unit (13), storage units (1, 11), a desulfurization unit (6, 12), a catalytic isomerization unit (9), and a gas scrubbing unit (7) are reused; and wherein the biorefinery is capable of :
(i) pretreatment of the lignocellulose biomasses provided as raw material with exclusively physical-mechanic methods to obtain hemicelluloses and celluloses;
(ii) saccharification of the hemicelluloses and of the celluloses to obtain soluble sugars;
(iii) separation of the insoluble lignin part;
(iv) performing fermentations and non-fermentation processes with the soluble sugars; and
(v) transforming of the lignins.

2. The method according to claim 1, **characterized in that** pretreatment (i) of the lignocellulose biomasses with steam explosion process at atmospheric pressure and/or, at a pressure lower than the atmospheric pressure, according to a process of the vacuum flash type, using the distillation column as flash vessel is performed by reusing a distillation column from the distillation or conversion unit (2 or 13) of the oil refinery.

3. The method according to claim 1, **characterized in that** at least some of the storage tanks (1, 11) of the former oil refinery are reused as fermentation reactors designed to perform mutually different fermentations according to step (iv).

4. The method according to one or more of the preceding claims, **characterized in that** at least one gas scrubbing unit (7) of the former oil refinery is reused to perform separation of carbon dioxide from the biogas produced by biomethane fermentations according to step (iv).

5. The method according to one or more of the preceding claims, **characterized in that** the desulfurization units (6, 12) and/or catalytic isomerization units (9) of the former oil refinery are reused to hydrogenate the soluble sugars resulting from step (ii).

6. The method according to one or more of the preceding claims, **characterized in that** the production units of the former oil refinery equipped with a fractionation column are reused in order to distill organic compounds obtained from the fermentations according to step (iv).

7. The method according to one or more of the preceding claims, **characterized in that** units for the self-generation of electric power and heat of the former oil refinery are reused in order to meet the enthalpic and electrical demands of the new processes.

## Patentansprüche

1. Ein Verfahren zur Herstellung integrierter Bioraffinerien von Lignocellulosebiomasse, wobei die Bioraffinerien die Lignocellulosebiomasse in Produkte zur lebensmittelbezogenen, industriellen, energiebezogenen und landwirtschaftlichen Verwendung umwandeln; **dadurch gekennzeichnet, dass**
Produktionseinheiten früherer Ölraffinerien, gewählt aus einer Destillationseinheit (2), einer Umwandlungseinheit (13), Speichereinheiten (1, 11), einer Entschwefelungseinheit (6, 12), einer katalytischen Isomerisationseinheit (9) und einer Gaswascheinheit (7) wiederverwendet werden; und wobei die Bioraffinerie zu Folgendem in der Lage ist:
(i) Vorbehandlung der Lignocellulosebiomasse, die als Rohmaterial bereitgestellt wird, mit ausschließlich physikochemischen Verfahren, um Hemicellulosen und Cellulosen zu erhalten;
(ii) Verzuckerung der Hemicellulosen und der Cellulosen, um lösliche Zucker zu erhalten;
(iii) Abtrennung des unlöslichen Ligninanteils;
(iv) Durchführen von Fermentationen und Nicht-Fermentationsvorgängen mit den löslichen Zuckern und
(v) Umwandeln der Lignine.

2. Das Verfahren gemäß Anspruch 1, **dadurch gekennzeichnet, dass** die Vorbehandlung (i) der Lignocellulosebiomasse mit einem Dampfexplosionsprozess bei atmosphärischem Druck und/oder bei einem Druck, der niedriger ist als der atmosphärische Druck, gemäß einem Verfahren vom Vacuum Flash-Typ, unter Verwendung der Destillationskolonne als Flashkammer, durch Wiederverwendung einer Destillationskolonne von der Destillations- oder Umwandlungseinheit (2 oder 13) der Ölraffinerie, durchgeführt wird.

3. Das Verfahren gemäß Anspruch 1, **dadurch gekennzeichnet, dass** mindestens einige der Lagertanks (1, 11) der früheren Ölraffinerie als Fermentationsreaktoren wiederverwendet werden, die konstruiert sind, um verschiedene Fermentationen entsprechend Schritt (iv) durchzuführen.

4. Das Verfahren gemäß einem oder mehreren der obigen Ansprüche, **dadurch gekennzeichnet, dass** mindestens eine Gaswascheinheit (7) der früheren Ölraffinerie wiederverwendet wird, um eine Trennung des Kohlendioxids vom Biogas durchzuführen, das durch Biomethan-Fermentationen gemäß Schritt (iv) erzeugt wurde.

5. Das Verfahren gemäß einem oder mehreren der obigen Ansprüche, **dadurch gekennzeichnet, dass** die Entschwefelungseinheiten (6, 12) und/oder katalytischen Isomerisationseinheiten (9) der früheren Ölraffinerie wiederverwendet werden, um die löslichen Zucker zu hydrieren, die aus Schritt (ii) resultieren.

6. Das Verfahren gemäß einem oder mehreren der obigen Ansprüche, **dadurch gekennzeichnet, dass** die Produktionseinheiten der früheren Ölraffinerie, die mit einer Fraktionierkolonne ausgestattet sind, wiederverwendet werden, um organische Verbindungen zu destillieren, die aus den Fermentationen gemäß Schritt (iv) gewonnen wurden.

7. Das Verfahren gemäß einem oder mehreren der obigen Ansprüche, **dadurch gekennzeichnet, dass** Einheiten zur Erzeugung von elektrischem Strom und Wärme der früheren Ölraffinierie wiederverwendet werden, um den enthalpischen und elektrischen Bedarf der neuen Verfahren zu decken.

## Revendications

1. Procédé pour obtenir des bioraffineries intégrées de biomasses lignocellulosiques, lesquelles bioraffineries transforment lesdites biomasses lignocellulosiques en produits pour une utilisation alimentaire, industrielle, liée à l'énergie et agricole, **caractérisé en ce que** :
des unités de production d'anciennes raffineries de pétrole choisies parmi une unité de distillation (2), une unité de conversion (13), des unités de stockage (1, 11), une unité de désulfuration (6, 12), une unité d'isomérisation catalytique (9) et une unité d'épuration des gaz (7) sont réutilisées ; et dans lequel la bioraffinerie est apte à :
(i) un prétraitement des biomasses lignocellulosiques fournies en tant que matière première exclusivement avec des procédés physiques et mécaniques pour obtenir des hémicelluloses et des celluloses ;
(ii) une saccharification des hémicelluloses et des celluloses pour obtenir des sucres solubles ;
(iii) une séparation de la part de lignine insoluble ;
(iv) l'exécution de processus de fermentation et de non fermentation avec les sucres solubles ; et
(v) la transformation des lignines.

2. Procédé selon la revendication 1, **caractérisé en ce que** le prétraitement (i) des biomasses lignocellulosiques avec un procédé d'explosion à la vapeur à la pression atmosphérique et/ou à une pression inférieure à la pression atmosphérique, selon un procédé du type éclair sous vide, en utilisant la colonne de distillation comme enceinte à éclair est réalisé en réutilisant une colonne de distillation de l'unité de distillation ou de conversion (2 ou 13) de la raffinerie de pétrole.

3. Procédé selon la revendication 1, **caractérisé en ce qu'**au moins une partie des réservoirs de stockage (1, 11) de l'ancienne raffinerie de pétrole est réutilisée comme réacteurs de fermentation pour réaliser des fermentations mutuellement différentes selon l'étape (iv).

4. Procédé selon l'une ou plusieurs des revendications précédentes, **caractérisé en ce qu'**au moins une unité d'épuration des gaz (7) de l'ancienne raffinerie de pétrole est réutilisée pour réaliser la séparation du dioxyde de carbone d'avec le biogaz produit par les fermentations de biométhane selon l'étape (iv).

5. Procédé selon l'une ou plusieurs des revendications précédentes, **caractérisé en ce que** les unités de désulfuration (6, 12) et/ou les unités d'isomérisation catalytique (9) de l'ancienne raffinerie de pétrole sont réutilisées pour hydrogéner les sucres solubles résultant de l'étape (ii).

6. Procédé selon l'une ou plusieurs des revendications précédentes, **caractérisé en ce que** les unités de production de l'ancienne raffinerie de pétrole équipées d'une colonne de fractionnement sont réutilisées afin de distiller des composés organiques obtenus à partir des fermentations de l'étape (iv).

7. Procédé selon l'une ou plusieurs des revendications précédentes, **caractérisé en ce que** des unités pour l'autoproduction d'énergie électrique et de chaleur de l'ancienne raffinerie de pétrole sont réutilisées afin de répondre aux besoins enthalpiques et électriques des nouveaux processus.
